# EUROPÄISCHE PATENTSCHRIFT

(11) **EP 0 632 053 B1**
(45) Veröffentlichungstag und Bekanntmachung des Hinweises auf die Patenterteilung: **08.09.1999**
(21) Anmeldenummer: 94104396.0
(22) Anmeldetag: 21.03.1994
(51) Int. Cl.: C07K 7/52, A61K 38/08, C07K 17/08

(54) **Cyclische Adhäsionsinhibitoren**
Cyclic adhesion inhibitors
Inhibiteurs d'adhésion cycliques

(30) Priorität: 01.04.1993 DE 4310643
(43) Veröffentlichungstag der Anmeldung: 04.01.1995
(73) Patentinhaber: MERCK PATENT GmbH, 64293 Darmstadt (DE)
(72) Erfinder: Jonczyk, Alfred, Dr., D-64295 Darmstadt (DE); Hölzemann, Günter, Dr., D-64342 Seeheim (DE); Felding-Habermann, Brunhilde, Dr., c/o BFH, Pgm of, 10666 N.Torrey Pines Rd,La Jolla,CA92037 (US); Rippmann, Friedrich, Dr., D-69120 Heidelberg (DE); Diefenbach, Beate, D-64289 Darmstadt (DE); Kessler, Horst, Prof. Dr., D-85748 Garching (DE); Haubner, Roland, D-85748 Garching (DE); Wermuth, Jochen, D-85748 Garching (DE)

(56) Entgegenhaltungen:
- EP-A- 0 578 083
- CHEMICAL ABSTRACTS, Band 119, Nr. 5, 2. August 1993, Columbus, Ohio, USA MUELLER; G.; GURRATH, M.; KURZ, M.; KESSLER H. " VI turns in peptides and proteins: A model peptide mimicry" Seite 1005, Spalte 2, Zusammenfassung Nr. 49 882d; & Proteins: Struct., Funct., Genet. 1993, 15(3), 235-51
- CHEMICAL ABSTRACTS, Band 118, Nr. 17, 26. April 1993, Columbus, Ohio, USA UENO, Y.; TAJIMA, M.; KUMAGAI, H. "Preparation of cyclic peptides and their salts as inhibitors of cell adhesion, tumors, and blood platelet aggregation" Seite 936, Spalte 1, Zusammenfassung Nr. 169 613t; & JP-A-04 264 097

## Beschreibung

Die Erfindung betrifft neue Cyclopeptide der Formel I

Cyclo-(Arg-B-Asp-X-Y) I,

worin
- B: Gly, Ala, -HN-Q-CO- und
- X und Y: jeweils unabhängig voneinander Gly, -HN-Q-CO-, Ala, Asn, Asp, Asp(OR), Arg, Cha, Cys, Gln, Glu, His, Ile, Leu, Lys, Lys(Ac), Lys(AcNH₂), Lys(AcSH), Met, Nal, Nle, Orn, Phe, 4-Hal-Phe, Phg, Pro, Pya, Ser, Thr, Tia, Tic, Trp, Tyr oder Val, wobei die genannten Aminosäurereste auch derivatisiert sein können,
wobei, falls X Phe bedeutet, Y nicht Val bedeutet,
- R: Alkyl mit 1-6 C-Atomen,
- Hal: F, Cl, Br, I,
- Q: Alkylen mit 1-6 C-Atomen und
- Ac: Alkanoyl mit 1-10 C-Atomen
bedeuten,
wobei, sofern es sich um Reste optisch aktiver Aminosäuren und Aminosäurederivate handelt, sowohl die D- als auch die L-Formen eingeschlossen sind, sowie deren physiologisch unbedenklichen Salze.

Ähnliche Verbindungen sind aus Pharmazie 40 (8), 532-5 (1985) bekannt.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze sehr wertvolle Eigenschaften besitzen. Vor allem wirken sie als Integrin-Inhibitoren, wobei sie insbesondere die Wechselwirkungen der β₃- oder β₅-Integrin-Rezeptoren mit Liganden hemmen. Besondere Wirksamkeit zeigen die Verbindungen im Fall der Integrine aᵥβ₃, aᵥβ₅ und a_{IIb}β₃. Diese Wirkung kann z.B. nach der Methode nachgewiesen werden, die von J.W. Smith et al. in J. Biol. Chem. 265, 12267-12271 (1990) beschrieben wird. Zusätzlich treten antiinflammatorische Effekte auf. Alle diese Wirkungen können mit Hilfe von literaturbekannten Methoden nachgewiesen werden.

Die Verbindungen können als Arzneimittelwirkstoffe in der Human- und Veterinärmedizin eingesetzt werden, insbesondere zur Prophylaxe und zur Behandlung von Erkrankungen des Kreislaufs, Thrombose, Herzinfarkt, Arteriosklerose, Entzündungen, Apoplexie, Angina pectoris, Tumorerkrankungen, osteolytischen Erkrankungen, insbesondere Osteoporose, Angiogenese und Restenose nach Angioplastie. Ferner können die Verbindungen zur Verbesserung der Wundheilung eingesetzt werden.

Die Verbindungen eignen sich zudem als antimikrobielle Wirkstoffe, die Infektionen, wie sie beispielsweise durch Bakterien, Pilze oder Hefen ausgelöst werden können, verhindern. Die Substanzen können daher vorzugsweise als begleitende antimikrobielle Wirkstoffe gegeben werden, wenn Eingriffe an Organismen vorgenommen werden, bei denen körperfremde Stoffe, wie z.B. Biomaterialien, Implantate, Katheter oder Herzschrittmacher, eingesetzt werden. Sie wirken als Antiseptika. Die vor- und nachstehend aufgeführten Abkürzungen von Aminosäureresten stehen für die Reste folgender Aminosäuren:
- Abu: 4-Aminobuttersäure
- Aha: 6-Aminohexansäure
- Ala: Alanin
- Asn: Asparagin
- Asp: Asparaginsäure
- Asp(OR): Asparaginsäure(β-ester)
- Arg: Arginin
- Cha: 3-Cyclohexylalanin
- Cit: Citrullin
- Cys: Cystein
- Dab: 2,4-Diaminobuttersäure
- Gln: Glutamin
- Glu: Glutaminsäure
- Gly: Glycin
- His: Histidin
- Ile: Isoleucin
- Leu: Leucin
- Lys: Lysin
- Lys(Ac): N^{ε}-Alkanoyllysin
- Lys(AcNH₂): N^{ε}-Aminoalkanoylysin
- Lys(AcSH): N^{ε}-Mercaptoalkanoyllysin
- Met: Methionin
- Nal: 3-(2-Naphthyl)-alanin
- Nle: Norleucin
- Orn: Ornithin
- Phe: Phenylalanin
- 4-Hal-Phe: 4-Halogen-phenylalanin
- Phg: Phenylglycin
- Pro: Prolin
- Pya: 3-(2-Pyridyl)-alanin
- Ser: Serin
- Tia: 3-(2-Thienyl)-alanin
- Tic: Tetrahydroisochinolin-3-carbonsäure
- Thr: Threonin
- Trp: Tryptophan
- Tyr: Tyrosin
- Val: Valin.

Ferner bedeuten nachstehend:
- BOC: tert-Butoxycarbonyl
- CBZ: Benzyloxycarbonyl
- DCCI: Dicyclohexylcarbodiimid
- DMF: Dimethylformamid
- EDCI: N-Ethyl-N'-(3-dimethylaminopropyl)-carbodiimidhydrochlorid
- Et: Ethyl
- Fmoc: 9-Fluorenylmethoxycarbonyl
- HOBt: 1-Hydroxybenzotriazol
- Me: Methyl
- Mtr: 4-Methoxy-2,3,6-trimethylphenyl-sulfonyl
- OBut: tert.-Butylester
- OMe: Methylester
- OEt: Ethylester
- POA: Phenoxyacetyl
- TBTU: 2-(1H-Benzotriazol-1-yl)-1,1,3,3-tetramethyluroniumtetrafluorborat
- TFA: Trifluoressigsäure.

Sofern die vorstehend genannten Aminosäuren in mehreren enantiomeren Formen auftreten können, so sind vor- und nachstehend, z.B. als Bestandteil der Verbindungen der Formel I, alle diese Formen und auch ihre Gemische (z.B. die DL-Formen) eingeschlossen. Ferner können die Aminosäuren, z.B. als Bestandteil von Verbindungen der Formel I, mit entsprechenden an sich bekannten Schutzgruppen versehen sein.

Gegenstand der Erfindung ist ferner ein Verfahren zur Herstellung einer Verbindung der Formel I nach Anspruch 1 oder eines ihrer Salze, dadurch gekennzeichnet, daß man sie aus einem ihrer funktionellen Derivate durch Behandeln mit einem solvolysierenden oder hydrogenolysierenden Mittel in Freiheit setzt
oder daß man ein Peptid der Formel II

H-Z-OH II

worin
- Z: -Arg-B-Asp-X-Y-
-B-Asp-X-Y-Arg-
-Asp-X-Y-Arg-B-
-X-Y-Arg-B-Asp- oder
-Y-Arg-B-Asp-X-bedeutet,
oder ein reaktionsfähiges Derivat eines solchen Peptids mit einem cyclisierenden Mittel behandelt
und/oder daß man eine basische oder saure Verbindung der Formel I durch Behandeln mit einer Säure oder Base in eines ihrer Salze überführt.

Vor- und nachstehend haben die Reste B, X, Y und Z die bei den Formeln I und II angegebenen Bedeutungen, sofern nicht ausdrücklich etwas anderes angegeben ist.

In den vorstehenden Formeln steht Alkyl vorzugsweise für Methyl, Ethyl, Isopropyl oder tert.-Butyl.

Die Gruppe B ist vorzugsweise Gly, aber auch -HN(-CH₂)₂-CO-, -HN-(CH₂)₃-CO-, -HN-(CH₂)₅-CO oder Ala. X ist vorzugsweise Phe, insbesondere D-Phe, aber auch 4-Hal-Phe, besonders 4-I-Phe sowie Pro, Tic, Lys, Nal oder Phg, wobei die D-Formen besonders bevorzugt sind.

Dementsprechend sind Gegenstand der Erfindung insbesondere diejenigen Verbindungen der Formel I, in denen mindestens einer der genannten Reste eine der vorstehend angegebenen bevorzugten Bedeutungen hat.

Eine bevorzugte Gruppe von Verbindungen kann durch die Teilformel Ia ausgedruckt werden, die sonst der Formel I entspricht, worin jedoch
- B: Gly, -HN-(CH₂)₂-CO-, -HN-(CH₂)₃-CO-, -HN-(CH₂)₅-CO- oder Ala
- X: D-Pro, D-Tic, Phe, D-Nal, D-Phg oder 4-I-Phe und
- Y: Val, Lys, Gly, Ala, Phe, Leu, Lys(Ac) oder Nle
bedeuten , wobei, falls X Phe bedeutet, Y nicht Val bedeutet.

Eine weitere bevorzugte Gruppe von Verbindungen kann durch die Teilformel Ib ausgedrückt werden, die sonst der Formel I entspricht, worin jedoch
- B: Gly,
- X: D-Phe und
- Y: Lys oder Gly
bedeuten.

Die Verbindungen der Formel I und auch die Ausgangsstoffe zu ihrer Herstellung werden im übrigen nach bekannten Methoden hergestellt, wie sie in der Literatur (z. B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

Die Verbindungen der Formel I können erhalten werden, indem man sie aus ihren funktionellen Derivaten durch Solvolyse, insbesondere Hydrolyse, oder durch Hydrogenolyse in Freiheit setzt.

Bevorzugte Ausgangsstoffe für die Solvolyse bzw. Hydrogenolyse sind solche, die anstelle einer oder mehrerer freier Amino- und/oder Hydroxygruppen entsprechende geschützte Amino- und/oder Hydroxygruppen enthalten, vorzugsweise solche, die anstelle eines H-Atoms, das mit einem N-Atom verbunden ist, eine Aminoschutzgruppe tragen, z. B. solche, die der Formel I entsprechen, aber anstelle einer NH₂-Gruppe eine NHR'-Gruppe (worin R' eine Aminoschutzgruppe bedeutet, z. B. BOC oder CBZ) enthalten.

Ferner sind Ausgangsstoffe bevorzugt, die anstelle des H-Atoms einer Hydroxygruppe eine Hydroxyschutzgruppe tragen, z. B. solche, die der Formel I entsprechen, aber anstelle einer Hydroxyphenylgruppe eine R''O-phenylgruppe enthalten (worin R'' eine Hydroxyschutzgruppe bedeutet).

Es können auch mehrere - gleiche oder verschiedene - geschützte Amino- und/oder Hydroxygruppen im Molekül des Ausgangsstoffes vorhanden sein. Falls die vorhandenen Schutzgruppen voneinander verschieden sind, können sie in vielen Fällen selektiv abgespalten werden.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an anderen Stellen des Moleküls duchgeführt worden ist. Typisch für solche Gruppen sind insbesondere unsubstituierte oder substituierte Acyl-, Aryl-, Aralkoxymethyl- oder Aralkylgruppen. Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren in weitestem Sinne aufzufassen. Er umschließt von aliphatischen, araliphatischen, aromatischen oder heterocyclischen Carbonsäuren oder Sulfonsäuren abgeleitete Arylgruppen sowie insbesondere Alkoxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie POA; Alkoxycarbonyl wie Methoxycarbonyl, Ethoxycarbonyl, 2,2,2-Trichlorethoxycarbonyl, BOC, 2-Jod-ethoxycarbonyl; Aralkyloxycarbonyl wie CBZ ("Carbobenzoxy"), 4-Methoxybenzyloxycarbonyl, FMOC; Arylsulfonyl wie Mtr. Bevorzugte Aminoschutzgruppen sind BOC und Mtr, ferner CBZ, Fmoc, Benzyl und Acetyl.

Der Ausdruck "Hydroxyschutzgruppe" ist ebenfalls allgemein bekannt und bezieht sich auf Gruppen, die geeignet sind, eine Hydroxygruppe vor chemischen Umsetzungen zu schützen, die aber leicht entfernbar sind, nachdem die gewünschte chemische Reaktion an anderen Stellen des Moleküls durchgeführt worden ist. Typisch für solche Gruppen sind die oben genannten unsubstituierten oder substituierten Aryl-, Aralkyl- oder Acylgruppen, ferner auch Alkylgruppen. Die Natur und Größe der Hydroxyschutzgruppen ist nicht kritisch, da sie nach der gewünschten chemischen Reaktion oder Reaktionsfolge wieder entfernt werden; bevorzugt sind Gruppen mit 1-20, insbesondere 1-10 C-Atomen. Beispiele für Hydroxyschutzgruppen sind u.a. Benzyl, p-Nitrobenzoyl, p-Toluolsulfonyl, tert.Butyl und Acetyl, wobei Benzyl und tert.Butyl besonders bevorzugt sind. Die COOH-Gruppen ins Asparaginsäure und Glutaminsäure werden bevorzugt in Form ihrer tert.-Butylester geschützt (z.B. Asp (OBut)).

Die als Ausgangsstoffe zu verwendenden funktionellen Derivate der Verbindungen der Formel I können nach üblichen Methoden der Aminosäure- und Peptidsynthese hergestellt werden, wie sie z. B. in den genannten Standardwerken und Patentanmeldungen beschrieben sind, z. B. auch nach der Festphasenmethode nach Merrifield (B.F. Gysin u. R.B. Merrifield, J. Am. Chem. Soc. 94, 3102ff. (1972)).

Das In-Freiheit-Setzen der Verbindungen der Formel I aus ihren funktionellen Derivaten gelingt - je nach der benutzten Schutzgruppe - z. B. mit starken Säuren, zweckmäßig mit TFA oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Die Anwesenheit eines zusätzlichen inerten Lösungsmittels ist möglich, aber nicht immer erforderlich. Als inerte Lösungsmittel eignen sich vorzugsweise organische, beispielsweise Carbonsäuren wie Essigsäure, Ether wie Tetrahydrofuran oder Dioxan, Amide wie DMF, halogenierte Kohlenwasserstoffe wie Dichlormethan, ferner auch Alkohole wie Methanol, Ethanol oder Isopropanol sowie Wasser. Ferner kommen Gemische der vorgenannten Lösungsmittel in Frage. TFA wird vorzugsweise im Überschuß ohne Zusatz eines weiteren Lösungsmittels verwendet, Perchlorsäure in Form eines Gemisches aus Essigsäure und 70%iger Perchlorsäure im Verhältnis 9 : 1. Die Reaktionstemperaturen für die Spaltung liegen zweckmäßig zwischen etwa 0 und etwa 50°, vorzugsweise arbeitet man zwischen 15 und 30° (Raumtemperatur).

Die Gruppen BOC, OBut und Mtr können z. B. bevorzugt mit TFA in Dichlormethan oder mit etwa 3 bis 5 n HCl in Dioxan bei 15-30° abgespalten werden, die FMOC-Gruppe mit einer etwa 5- bis 50%igen Lösung von Dimethylamin, Diethylamin oder Piperidin in DMF bei 15-30°.

Hydrogenolytisch entfernbare Schutzgruppen (z. B. CBZ oder Benzyl) können z. B. durch Behandeln mit Wasserstoff in Gegenwart eines Katalysators (z. B. eines Edelmetallkatalysators wie Palladium, zweckmäßig auf einem Träger wie Kohle) abgespalten werden. Als Lösungsmittel eignen sich dabei die oben angegebenen, insbesondere z. B. Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt. Eine Hydrogenolyse der CBZ-Gruppe gelingt z. B. gut an 5 bis 10%igem Pd-C in Methanol oder mit Ammoniumformiat (anstelle von H₂) an Pd-C in Methanol/DMF bei 20-30°.

Verbindungen der Formel I können auch durch Cyclisierung von Verbindungen der Formel II unter den Bedingungen einer Peptidsynthese erhalten werden. Dabei arbeitet man zweckmäßig nach üblichen Methoden der Peptid-Synthese, wie sie z. B. in Houben-Weyl, 1.c., Band 15/II, Seiten 1 bis 806 (1974) beschrieben sind.

Die Reaktion gelingt vorzugsweise in Gegenwart eines Dehydratisierungsmittels, z. B. eines Carbodiimids wie DCCI oder EDCI, ferner Propanphosphonsäureanhydrid (vgl. Angew.Chem. 92, 129 (1980)), Diphenylphosphorylazid oder 2-Ethoxy-N-ethoxycarbonyl-1,2-dihydrochinolin, in einem inerten Lösungsmittel, z. B. einem halogenierten Kohlenwasserstoff wie Dichlormethan, einem Ether wie Tetrahydrofuran oder Dioxan, einem Amid wie DMF oder Dimethylacetamid, einem Nitril wie Acetonitril, oder in Gemischen dieser Lösungsmittel, bei Temperaturen zwischen etwa -10 und 40, vorzugsweise zwischen 0 und 30°. Um die intramolekulare Cyclisierung vor der intermolekularen Peptid-Bindung zu fördern, ist es zweckmäßig, in verdünnten Lösungen zu arbeiten (Verdünnungsprinzip).

Anstelle von II können auch geeignete reaktionsfähige Derivate dieser Stoffe in die Reaktion eingesetzt werden, z.B. solche, in denen reaktive Gruppen intermediär durch Schutzgruppen blockiert sind. Die Aminosäurederivate II können z.B. in Form ihrer aktivierten Ester verwendet werden, die zweckmäßig in situ gebildet werden, z. B. durch Zusatz von HOBt oder N-Hydroxysuccinimid.

Die Ausgangsstoffe der Formel II sind in der Regel neu. Sie können nach bekannten Methoden, z. B. den oben angegebenen Methoden der Peptidsynthese und der Abspaltung von Schutzgruppen, hergestellt werden.

In der Regel synthetisiert man zunächst geschützte Pentapeptidester der Formel R'-Z-OR'', z. B. BOC-Z-OMe oder BOC-Z-OEt, die zunächst zu Säuren der Formel R'-Z-OH, z. B. BOC-Z-OH verseift werden; aus diesen wird die Schutzgruppe R' abgespalten, wodurch man die freien Peptide der Formel H-Z-OH (II) erhält.

Eine Base der Formel I kann mit einer Säure in das zugehörige Säureadditionssalz übergeführt werden. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2- oder 3-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono-und -disulfonsäuren, Laurylschwefelsäure. Salze mit physiologisch nicht unbedenklichen Säuren, z.B. Pikrate, können zur Isolierung und/oder Aufreinigung der Verbindungen der Formel I verwendet werden.

Andererseits kann eine Säure der Formel I durch Umsetzung mit einer Base in eines ihrer physiologisch unbedenklichen Metall- oder Ammoniumsalze übergeführt werden. Als Salze kommen dabei insbesondere die Natrium-, Kalium-, Magnesium-, Calcium- und Ammoniumsalze in Betracht, ferner substituierte Ammoniumsalze, z.B. die Dimethyl-, Diethyl- oder Diisopropylammoniumsalze, Monoethanol-, Diethanol- oder Triethanolammoniumsalze, Cyclohexyl-, Dicyclohexylammoniumsalze, Dibenzylethylendiammonimsalze, weiterhin z.B. Salze mit N-Methyl-D-glucamin oder mit Arginin oder Lysin.

Die neuen Verbindungen der Formel I und ihre physiologisch unbedenklichen Salze können zur Herstellung pharmazeutischer Präparate verwendet werden, indem man sie zusammen mit mindestens einem Träger- oder Hilfsstoff und, falls erwünscht, zusammen mit einem oder mehreren weiteren Wirkstoff(en) in eine geeignete Dosierungsform bringt Die so erhaltenen Zubereitungen können als Arzneimittel in der Human- oder Veterinärmedizin eingesetzt werden. Als Trägersubstanzen kommen organische oder anorganische Stoffe in Frage, die sich für die enterale (z.B. orale oder rektale), parenterale (z.B. intravenöse Injektion) oder lokale (z.B. topische, dermale, ophthalmische oder nasale) Applikation oder für eine Applikation in Form eines Inhalations-Sprays eignen und mit den neuen Verbindungen nicht reagieren, beispielsweise Wasser oder wässerige isotonische Kochsalzslösung, niedere Alkohole, pflanzliche Öle, Benzylalkohole, Polyethylenglykole, Glycerintriacetat und andere Fettsäureglyceride, Gelatine, Sojalecithin, Kohlehydrate wie Lactose oder Stärke, Magnesiumstearat, Talk, Cellulose, Vaseline. Zur oralen Anwendung dienen insbesondere Tabletten, Dragees, Kapseln, Sirupe, Säfte oder Tropfen; von Interesse sind speziell Lacktabletten und Kapseln mit magensaftresistenten Überzügen bzw. Kapselhüllen. Zur rektalen Anwendung dienen Suppositorien, zur parenteralen Applikation Lösungen, vorzugsweise ölige oder wässerige Lösungen, ferner Suspensionen, Emulsionen oder Implantate. Zur topischen Anwendung eignen sich z.B. Lösungen, die in Form von Augentropfen verwendet werden können, ferner z. B. Suspensionen, Emulsionen, Cremes, Salben oder Komprimate. Für die Applikation als Inhalations-Spray können Sprays verwendet werden, die den Wirkstoff entweder gelöst oder suspendiert in einem Treibgas oder Treibgasgemisch (z.B. CO₂ oder Fluorchlorkohlenwasserstoffen) enthalten. Zweckmäßig verwendet man den Wirkstoff dabei in mikronisierter Form, wobei ein oder mehrere zusätzliche physiologisch verträgliche Lösungsmittel zugegen sein können, z.B. Ethanol. Inhalationslösungen können mit Hilfe üblicher Inhalatoren verabfolgt werden. Die neuen Verbindungen können auch lyophilisiert und die erhaltenen Lyophilisate z.B. zur Herstellung von Injektionspräparaten verwendet werden. Die Injektionen können dabei als Bolus oder als kontinuierliche Infusion (z.B. intravenös, intramusculär, subcutan oder intrathecal) gegeben werden. Die angegebenen Zubereitungen können sterilisiert sein und/oder Hilfsstoffe wie Konservierungs-, Stabilisierungs- und/oder Netzmittel, Emulgatoren, Salze zur Beeinflussung des osmotischen Druckes, Puffersubstanzen, Farb- und/oder Aromastoffe enthalten. Sie können, falls erwünscht, auch einen oder mehrere weitere Wirkstoffe enthalten, z. B. ein oder mehrere Vitamine.

Die erfindungsgemäßen Substanzen können in der Regel in Analogie zu anderen bekannten, im Handel befindlichen Peptiden, insbesondere aber in Analogie zu den in der US-A-4 472 305 beschriebenen Verbindungen verabreicht werden, vorzugsweise in Dosierungen zwischen etwa 0,05 und 500, insbesondere zwischen 0,5 und 100 mg pro Dosierungseinheit. Die tägliche Dosierung liegt vorzugsweise zwischen etwa 0,01 und 2 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die parenterale Applikation ist bevorzugt.

Ferner können die neuen Verbindungen der Formel I als Integrinliganden zur Herstellung von Säulen für die Affinitätschromatographie zur Reindarstellung von Integrinen verwendet werden.

Der Ligand, d.h. ein Peptidderivat der Formel I, wird dabei über Ankerfunktionen an einen polymeren Träger kovalent gekuppelt.

Als polymere Trägermaterialien eignen sich die an sich in der Peptidchemie bekannten polymeren festen Phasen mit vorzugsweise hydrophilen Eigenschaften, beispielsweise quervernetzte Polyzucker, wie Zellulose, Sepharose oder Sephadex®, Acrylamide, Polymer auf Polyethylenglykolbasis oder Tentakelpolymere®

Als Ankerfunktionen, die mit den polymeren Trägern verknüpft sind, eignen sich vorzugsweise lineare Alkylenketten mit 2-12 C-Atomen, die mit einem Ende direkt an das Polymer gebunden sind und am anderen Ende eine funktionelle Gruppe, wie z.B. Hydroxy, Amino, Mercapto, Maleinimido oder -COOH aufweisen und dazu geeignet sind, mit dem C- oder N-terminalen Abschnitt des jeweiligen Peptids verknüpft zu werden.

Dabei ist es möglich, daß das Peptid direkt oder ebenfalls über eine zweite Ankerfunktion mit dem Anker des Polymers verbunden ist. Ferner ist es möglich, daß Peptide, die Aminosäurereste mit funktionalisierten Seitenketten enthalten, über diese mit der Ankerfunktion des Polymers verbunden werden.

Darüber hinaus können bestimmte Aminosäurereste, die Bestandteil der Peptide der Formel I sind, in ihren Seitenketten erart modifiziert werden, so daß sie zur Verankerung über z.B. SH-, OH-, NH₂ oder COOH-Gruppen mit dem Anker des Polymers zur Verfügung stehen.

Möglich sind hierbei ungewöhnliche Aminosäuren, wie z.B. Phenylalaninderivate, die in 4-Position des Phenylrings eine Mercapto-, Hydroxy-, Amino- oder Carboxyalkylkette tragen, wobei die funktionelle Gruppe sich am Ende der Kette befindet.

Beispiele für Aminosäurereste, deren Seitenkette direkt als Ankerfunktion dienen kann, sind z.B. Lys, Orn, Arg, Dab, Asp, Asn, Glu, Gln, Ser, Thr, Cys, Cit oder Tyr.

Beispiele für N-terminale Anker sind Reste wie z.B. -CO-CₙH₂ₙ-NH₂, -CO-CₙH₂ₙ-OH, -CO-CₙH₂ₙ-SH oder -CO-CₙH₂ₙ-COOH mit n = 2-12, wobei die Länge der Alkylenkette nicht kritisch ist und diese gegebenenfalls auch z.B. durch entsprechende Aryl- oder Alkylarylreste ersetzt werden kann.

C-terminale Anker können beispielsweise -O-CₙH₂ₙ-SH, -O-CₙH₂ₙ-OH, -O-CₙH₂ₙ-NH₂, -O-CₙH₂ₙ-COOH, -NH-CₙH₂ₙ-SH, -NH-CₙH₂ₙ-OH, -NH-CₙH₂ₙ-NH₂ oder -NH-CₙH₂ₙ-COOH sein, wobei für n sowie die Alkylenkette das bereits im vorhergehenden Abschnitt Gesagte gilt.

Die N- und C-terminalen Anker können auch als Ankerbaustein für eine bereits funktionalisierte Seitenkette eines Aminosäurerests dienen. Es kommen hier beispielsweise Aminosäurereste wie Lys(CO-C₅H₁₀-NH₂), Asp(NH-C₃H₆-COOH) oder Cys(C₃H₆-NH₂) in Frage, wobei der Anker immer an die funktionelle Gruppe der Seitenkette gebunden ist.

Die Herstellung der Materialien für die Affinitätschromatographie zur Integrinreinigung erfolgt unter Bedingungen wie sie für die Kondensation von Aminosäuren üblich und an sich bekannt sind und bereits im Abschnitt zur Herstellung der Verbindungen der Formel I geschildert wurden.

Vor- und nachstehend sind alle Temperaturen in °C angegeben. In den nachfolgenden Beispielen bedeutet "übliche Aufarbeitung": Man gibt, falls erforderlich, Wasser hinzu, neutralisiert, extrahiert mit Ether oder Dichlormethan, trennt ab, trocknet die organische Phase über Natriumsulfat, filtriert, dampft ein und reinigt durch Chromatographie an Kieselgel und/oder Kristallisation. RZ = Retentionszeit (Minuten) bei HPLC an Lichrosorb® RP select B (250-4,7 µm)-Säule, Laufmittel: 0,3 % TFA in Wasser; Isopropanolgradient von 0-80 Vol% in 50 Min. bei 1 ml/Min. Fluß und Detektion bei 215 nm. M⁺ = Molekular-Peak im Massenspektrum, erhalten nach der "Fast Atom Bombardment"-Methode (FAB).

### Beispiel 1

Eine Lösung von 0,2 g H-Arg-Gly-Asp-D-Pro-Val-ONa [z.B. erhältlich aus Fmoc-Arg-Gly-Asp-D-Pro-Val-O-Wang, wobei -O-Wang den bei den modifizierten Merrifield-Techniken verwendeten Rest eines 4-Oxymethyl-phenoxymethyl-polystyrolharzes bedeutet, durch Abspaltung der Fmoc-Gruppe mit Piperidin/DMF und Abspaltung des Harzes mit TFA/CH₂Cl₂ (1:1)] in 15 ml DMF wird mit 85 ml Dichlormethan verdünnt und mit 50 mg NaHCO₃ versetzt. Nach Kühlung in einer Trockeneis/Aceton-Mischung werden 40 µl Diphenylphosphorylazid zugegeben. Nach 16 Stunden Stehen bei Raumtemperatur engt man die Lösung ein. Das Konzentrat wird gelfiltriert (Sephadex G10-Säule in Isopropanol/Wasser 8:2) und dann wie üblich mittels HPLC gereinigt. Man erhält Cyclo-(Arg-Gly-Asp-D-Pro-Val); RZ = 13,4; M⁺ 525.

Analog erhält man durch Cyclisierung der entsprechenden linearen Peptide:
Cyclo-(Arg-Gly-Asp-D-Tic-Val); RZ = 18,4; M⁺ 587;
Cyclo-(Arg-Gly-Asp-D-Lys-Val); RZ = 6,8; M⁺ 556;
Cyclo-(Arg-Gly-Asp-D-Phe-Lys); RZ = 10,9; M⁺ 604;
Cyclo-(Arg-Gly-Asp-D-Phe-Gly); RZ = 13,1; M⁺ 533;
Cyclo-(Arg-Gly-Asp-D-Phe-Ala); RZ = 14,3; M⁺ 547;
Cyclo-(Arg-Gly-Asp-D-Phe-Phe); RZ = 22,0; M⁺ 623;
Cyclo-(Arg-Gly-Asp-D-Phe-Leu); RZ = 21.2; M⁺ 589;
Cyclo-(Arg-Gly-Asp-D-Nal-Val); RZ = 24,7; M⁺ 625;
Cyclo-(Arg-Gly-Asp-D-Phg-Val); RZ = 16,5; M⁺ 561;
Cyclo-(Arg-Gly-Asp-Phe-Gly); RZ = 13,2; M⁺ 533;
Cyclo-(Arg-Gly-Asp-Phe-D-Ala); RZ = 14,8; M⁺ 547;
Cyclo-(Arg-Gly-Asp-Phe-D-Phe); RZ = 20,2; M⁺ 623;
Cyclo-(Arg-Gly-Asp-Phe-D-Leu); RZ = 21,4; M⁺ 589;
Cyclo-(Arg(Mtr)-Gly-Asp-D-Phe-Lys); RZ = 23,4; M⁺ 816;
Cyclo-(Arg-Gly-Asp-D-Phe-Lys(H₃C-CO); RZ = 17,0; M⁺ 646;
Cyclo-(Arg-Gly-Asp-D-Phe-Nle); RZ = 22,0; M⁺ 589;
Cyclo-(Arg-Gly-D-Asp-D-Phe-D-Gly); RZ = 18,7; M⁺ 575;
Cyclo-(Arg-Gly-Asp-D-(4-I-Phe)-Val); RZ = 23,3; M⁺ 701;
Cyclo-(Arg-Gly-Asp-Phe-D-Nal);
Cyclo-(Arg-Gly-Asp-Phe-D-Leu);
Cyclo-(Arg-Gly-Asp-Phe-D-Ser);
Cyclo-(Arg-Gly-Asp-D-Nal-Leu);
Cyclo-(Arg-Gly-Asp-Nal-D-Val);
Cyclo-(Arg-Gly-Asp-Phg-D-Val);
Cyclo-(Arg-Gly-Asp-Trp-D-Val);
Cyclo-(Arg-Gly-Asp-Ala(2-Thienyl)-D-Val);
Cyclo-(Arg-Gly-Asp-Lys-D-Val);
Cyclo-(Arg-Gly-Asp-(4-NO₂-Phe)-D-Val);
Cyclo-(Arg-Gly-Asp-Cha-D-Val);
Cyclo-(Arg-Gly-Asp-D-Phe-β-Ala);
Cyclo-(Arg-Gly-Asp-D-Phe-Abu);
Cyclo-(Arg-Gly-Asp-D-Phe-Aha);
Cyclo-(Arg-Gly-Asp-D-Phe-Lys);
Cyclo-(Arg-Gly-Asp-D-Phe-Pro);
Cyclo-(Arg-Gly-Asp-D-Phe-Arg);
Cyclo-(Arg-Gly-Asp-D-Phe-Lys(Aha));
Cyclo-(Arg-Gly-Asp-D-Phe-Nal);
Cyclo-(Arg-Gly-Asp-D-Phe-Ser);
Cyclo-(Arg(Mtr)-Gly-Asp-Phe-D-Nal);
Cyclo-(Arg(Mtr)-Gly-Asp-Phe-D-Leu);
Cyclo-(Arg(Mtr)-Gly-Asp-Phe-D-Ser);
Cyclo-(Arg(Mtr)-Gly-Asp-D-Nal-Leu);
Cyclo-(Arg(Mtr)-Gly-Asp-Nal-D-Val);
Cyclo-(Arg(Mtr)-Gly-Asp-Phg-D-Val);
Cyclo-(Arg(Mtr)-Gly-Asp-Trp-D-Val);
Cyclo-(Arg-Gly-Asp-D-Phe-Lys(BOC-Aha);
Cyclo-(Arg-Gly-Asp-D-Phe-Lys(CO-CH₂SH));
Cyclo-(Arg-Gly-Asp-D-Phe-Lys(CO-(CH₂)₂SH));
Cyclo-(Arg-Gly-Asp-D-Phe-Lys(CO-(CH₂)₃SH));
Cyclo-(Arg-Gly-Asp-D-Phe-Lys(CO-(CH₂)₄SH));
Cyclo-(Arg-Gly-Asp-D-Phe-Lys(CO-(CH₂)₅SH));
Cyclo-(Arg-Gly-Asp-D-Tyr-Val);
Cyclo-(Arg-Gly-Asp-D-(4-Cl-Phe)-Val);
Cyclo-(Arg-Gly-Asp-D-Pya-Val);
Cyclo-(Arg-Gly-Asp-D-NMe-Phe-Val);
Cyclo-(Arg-Gly-Asp-D-Tyr(OEt)-Val);
Cyclo-(Arg-Gly-Asp-D-Arg-Val);
Cyclo-(Arg-Gly-Asp-D-Trp-Val);
Cyclo-(Arg-Gly-Asp-D-Ala(2-Thienyl)-Val);
Cyclo-(Arg-Gly-Asp-D-Tyr-NMe-Val);

### Beispiel 2

Eine Lösung von 0,41 g Cyclo-(Arg(Mtr)-Gly-Asp-D-Phe-Lys) [erhältlich durch Cyclisierung gemäß Bsp.1] in 8,4 ml TFA, 1,7 ml Dichlormethan und 0,9 ml Thiophenol wird 4 Stunden bei Raumtemperatur stehen gelassen, anschließend eingeengt und nach Verdünnen mit Wasser gefriergetrocknet. Gelfiltration an Sephadex G 10 (Essigsäure/Wasser 1:1) und anschließende Reinigung durch präparative HPLC unter den angegebenen Bedingungen liefern Cyclo-(Arg-Gly-Asp-D-Phe-Lys) RZ = 10,9 M⁺ 604.

Analog erhält man:
aus Cyclo-(Arg(Mtr)-Gly-Asp-Phe-D-Nal):
   Cyclo-(Arg-Gly-Asp-Phe-D-Nal);
aus Cyclo-(Arg(Mtr)-Gly-Asp-Phe-D-Leu):
   Cyclo-(Arg-Gly-Asp-Phe-D-Leu);
aus Cyclo-(Arg(Mtr)-Gly-Asp-Phe-D-Ser):
   Cyclo-(Arg-Gly-Asp-Phe-D-Ser);
aus Cyclo-(Arg(Mtr)-Gly-Asp-D-Nal-Leu):
   Cyclo-(Arg-Gly-Asp-D-Nal-Leu);
aus Cyclo-(Arg(Mtr)-Gly-Asp-Nal-D-Val):
   Cyclo-(Arg-Gly-Asp-Nal-D-Val);
aus Cyclo-(Arg(Mtr)-Gly-Asp-Phg-D-Val):
   Cyclo-(Arg-Gly-Asp-Phg-D-Val);
aus Cyclo-(Arg(Mtr)-Gly-Asp-Trp-D-Val):
   Cyclo-(Arg-Gly-Asp-Trp-D-Val).

### Beispiel 3

80 mg Cyclo-(Arg-Gly-Asp-D-Phe-Leu) werden fünf- bis sechsmal in 0,01 m HCl gelöst und nach jedem Lösevorgang gefriergetrocknet. Anschließende Reinigung durch HPLC liefert Cyclo-(Arg-Gly-Asp-D-Phe-Leu) x HCl; RZ = 20,6; M⁺ 589.

Analog erhält man
aus Cyclo-(Arg-Gly-Asp-D-Phe-Leu):
   Cyclo-(Arg-Gly-Asp-D-Phe-Leu) x HCl;
aus Cyclo-(Arg-Gly-Asp-D-Phe-Leu) durch Behandlung mit Essigsäure:
   Cyclo-(Arg-Gly-Asp-D-Phe-Leu) x H₃C-COOH; RZ = 19,2; M⁺ 589;
aus Cyclo-(Arg-Gly-Asp-D-Phe-Leu) durch Behandlung mit Salpetersäure:
   Cyclo-(Arg-Gly-Asp-D-Phe-Leu) x HNO₃; RZ = 20,4; M⁺ 589;

### Beispiel 4

Zur Herstellung von Affinitätsphasen suspendiert man 0,9g N-Maleinimido-(CH₂)₅-CO-NH-(CH₂)₃-Polymer [erhältlich durch Kondensation von N-Maleinimido-(CH₂)₅-COOH mit H₂N-(CH₂)₃-Polymer] in 10 ml 0,1 M Natriumphosphatpuffer bei pH 7 und fügt bei 4° 1 Äquivalent Cyclo-(Arg-Gly-Asp-D-Phe-Lys(CO(CH₂)₂SH) hinzu. Man rührt 4 Stunden bie gleichzeitiger Erwärmung der Reaktionsmischung auf Raumtemperatur, filtriert den festen Rückstand ab und wäscht zweimal mit je 10 ml Pufferlösung (pH 7) und anschließend dreimal mit je 10 ml Wasser. Man erhält Cyclo-(Arg-Gly-Asp-D-Phe-Lys(CO(CH₂)₂S-3-(N-maleinimido-(CH₂)₅-CONH-(CH₂)₃-Polymer)).

### Beispiel 5

Analog Beispiel 1 erhält man durch Kondensation von Polymer-O-(CH₂)₃-NH₂ [im Handel erhältlich] und Cyclo-(Arg-Gly-Asp-D-Pro-Lys(CO(CH₂)₄COOH) [erhältlich durch Kondensation von Adipinsäure mit Cyclo-(Arg-Gly-Asp-D-Pro-Lys) unter den genannten Bedingungen] die folgende polymere Phase: Cyclo-(Arg-Gly-Asp-D-Pro-Lys-(CO-(CH₂)₄-CO-NH-(CH₂)₃-O-Polymer)

Analog erhält man durch Kondensation von

Cyclo-(Arg-Gly-Asp-D-Phe-Lys-(CO-(CH₂)₅-NH₂)) mit HOOC-CH₂-O-Polymer:
Cyclo-(Arg-Gly-Asp-D-Phe-Lys-(CO-(CH₂)₅-NH-CO-CH₂-O-Polymer)).

Die nachstehenden Beispiele betreffen pharmazeutische Zubereitungen.

### Beispiel A: Injektionsgläser

Eine Lösung von 100 g eines Cyclopeptids der Formel I und 5 g Dinatriumhydrogenphosphat in 3 l zweifach destilliertem Wasser wird mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g Wirkstoff der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g Wirkstoff der Formel I, 9,38 g NaH₂PO₄ x 2 H₂O, 28,48 g Na₂HPO₄ x 12 H₂O und 0,1 g Benzalkoniumchlorid in 940 ml zweifach destilliertem Wasser. Man stellt auf pH 6,8 ein, füllt auf 1 l auf und sterilisiert durch Bestrahlung. Diese Lösung kann in Form von Augentropfen verwendet werden.

### Beispiel D: Salbe

Man mischt 500 mg Wirkstoff der Formel I mit 99,5 g Vaseline unter aseptischen Bedingungen.

### Beispiel E: Tabletten

Ein Gemisch von 100 g eines Cyclopeptids der Formel I, 1 kg Lactose, 600 g mikrokristalliner Cellulose, 600 g Maisstärke, 100 g Polyvinylpyrrolidon, 80 g Talk und 10 g Magnesiumstearat wird in üblicher Weise zu Tabletten gepreßt, so das jede Tablette 10 mg Wirkstoff enthält.

### Beispiel F: Dragees

Man preßt Tabletten wie in Beispiel E angegeben und überzieht sie anschließend in üblicher Weise mit einem Überzug aus Saccharose, Maisstärke, Talk, Tragant und Farbstoff.

### Beispiel G: Kapseln

In üblicher Weise werden Hartgelatinekapseln mit einem Wirkstoff der Formel I gefüllt, so daß jede Kapsel 5 mg Wirkstoff enthält.

### Beispiel H: Inhalationsspray

Man löst 14 g Wirkstoff der Formel I in 10 l isotonischer NaCl-Lösung und füllt die Lösung in handelsübliche Sprühgefäße mit Pump-Mechanismus. Die Lösung kann in Mund oder Nase gesprüht werden. Ein Sprühstoß (etwa 0,1 ml) entspricht einer Dosis von etwa 0,14 mg.

## Patentansprüche

1. Cyclopeptide der Formel I
Cyclo-(Arg-B-Asp-X-Y) I
worin
B Gly, Ala, -HN-Q-CO- und
X und Y jeweils unabhängig voneinander Gly, -HN-Q-CO-, Ala, Asn, Asp, Asp(OR), Arg, Cha, Cys, Gln, Glu, His, Ile, Leu, Lys, Lys(Ac), Lys(AcNH₂), Lys(AcSH), Met, Nal, Nle, Orn, Phe, 4-Hal-Phe, Phg, Pro, Pya, Ser, Thr, Tia, Tic, Trp, Tyr oder Val, wobei die genannten Aminosäurereste auch derivatisiert sein können,
wobei, falls X Phe bedeutet, Y nicht Val bedeutet,
R Alkyl mit 1-6 C-Atomen,
Hal F, Cl, Br, I,
Q Alkylen mit 1-6 C-Atomen und
Ac Alkanoyl mit 1-10 C-Atomen bedeuten,
wobei, sofern es sich um Reste optisch aktiver Aminosäuren und Aminosäurederivate handelt, sowohl die D- als auch die L-Formen eingeschlossen sind, sowie deren physiolgisch unbedenklichen Salze.

2. Ein Enantiomer oder ein Diastereomer einer Verbindung der Formel I gemäß Anspruch 1.

3. (a) Cyclo-(Arg-Gly-Asp-D-Lys-Val);
(b) Cyclo-(Arg-Gly-Asp-D-Phe-Lys);
(c) Cyclo-(Arg-Gly-Asp-D-Phe-Gly);
(d) Cyclo-(Arg-Gly-Asp-D-Phe-Phe);
(e) Cyclo-(Arg-Gly-Asp-D-Phe-Leu);
(f) Cyclo-(Arg-Gly-Asp-Phe-D-Leu).

4. Verfahren zur Herstellung einer Verbindung der Formel I nach Anspruch 1 oder eines ihrer Salze, daurch gekennzeichnet, daß man sie aus einem ihrer funktionellen Derivate durch Behandeln mit einem solvolysierenden oder hydrogenolysierenden Mittel in Freiheit setzt,
oder daß man ein Peptid der Formel II
H-Z-OH II
worin
Z -Arg-B-Asp-X-Y-
-B-Asp-X-Y-Arg-
-Asp-X-Y-Arg-B-
-X-Y-Arg-B-Asp- oder
-Y-Arg-B-Asp-X- bedeutet,
oder ein reaktionsfähiges Derivat eines solchen Peptids mit einem cyclisierenden Mittel behandelt.

5. Verfahren zur Herstellung pharmazeutischer Zubereitungen, dadurch gekennzeichnet, daß man eine Verbindung der Formel I nach Anspruch 1 und/oder eines ihrer physiologisch unbedenklichen Salze zusammen mit mindestens einem festen, flüssigen oder halbflüssigen Träger- oder Hilfsstoff in eine geeignete Dosierungsform bringt.

6. Pharmazeutische Zubereitung, gekennzeichnet durch einen Gehalt an mindestens einer Verbindung der allgemeinen Formel I nach Anspruch 1 und/oder einem ihrer physiologisch unbedenklichen Salze.

7. Verwendung von Verbindungen der Formel I nach Anspruch 1 oder von deren physiologisch unbedenklichen Salzen zur Herstellung eines Arzneimittels zur Bekämpfung von Krankheiten.

8. Verbindungen der Formel I nach Anspruch 1 oder von deren physiologisch unbedenklichen Salzen bei zur Verwendung bei der Bekämpfung von Krankheiten.

9. Verwendung von Verbindungen der Formel I nach Anspruch 1 zur Herstellung von immobilisierten Liganden für Affinitätssäulenchromatographie.

10. Verwendung von Verbindungen der Formel I nach Anspruch 1 zur Reinigung von Integrinen durch Affinitätschromatographie.

## Claims

1. Cyclopeptides of the formula I
cyclo-(Arg-B-Asp-X-Y) I
in which
B is Gly, Ala, -HN-Q-CO- and
X and Y in each case independently of one another are Gly, -HN-Q-CO-, Ala, Asn, Asp, Asp(OR), Arg, Cha, Cys, Gln, Glu, His, Ile, Leu, Lys, Lys(Ac), Lys(AcNH₂), Lys(AcSH), Met, Nal, Nle, Orn, Phe, 4-Hal-Phe, Phg, Pro, Pya, Ser, Thr, Tia, Tic, Trp, Tyr or Val, where the said amino acid radicals can also be derivatized, where, if X is Phe, Y is not Val,
R is alkyl having 1-6 C atoms,
Hal is F, Cl, Br or I,
Q is alkylene having 1-6 C atoms and
Ac is alkanoyl having 1-10 C atoms,
where, if it is a case of radicals of optically active amino acids and amino acid derivatives, both the D and the L forms are included, and also their physiologically acceptable salts.

2. An enantiomer or a diastereomer of a compound of the formula I according to Claim 1.

3. (a) cyclo-(Arg-Gly-Asp-D-Lys-Val);
(b) cyclo-(Arg-Gly-Asp-D-Phe-Lys);
(c) cyclo-(Arg-Gly-Asp-D-Phe-Gly);
(d) cyclo-(Arg-Gly-Asp-D-Phe-Phe);
(e) cyclo-(Arg-Gly-Asp-D-Phe-Leu); (f) cyclo-(Arg-Gly-Asp-Phe-D-Leu).

4. Process for the preparation of a compound of the formula I according to Claim 1 or one of its salts, characterized in that it is liberated from one of its functional derivatives by treating with a solvolysing or hydrogenolysing agent,
or in that a peptide of the formula II
H-Z-OH II
in which
Z is -Arg-B-Asp-X-Y-
-B-Asp-X-Y-Arg-
-Asp-X-Y-Arg-B-
-X-Y-Arg-B-Asp- or
-Y-Arg-B-Asp-X-,
or a reactive derivative of such a peptide is treated with a cyclizing agent.

5. Method for the production of pharmaceutical preparations, characterized in that a compound of the formula I according to Claim 1 and/or one of its physiologically acceptable salts are brought into a suitable dosage form together with at least one solid, liquid or semisolid excipient or auxiliary.

6. Pharmaceutical preparation, characterized in that it contains at least one compound of the general formula I according to Claim 1 and/or one of its physiologically acceptable salts.

7. Use of compounds of the formula I according to Claim 1 or their physiologically acceptable salts for the production of a medicament for the control of diseases.

8. Compounds of the formula I according to Claim 1 or their physiologically acceptable salts for use in the control of diseases.

9. Use of compounds of the formula I according to Claim 1 for the production of immobilized ligands for affinity column chromatography.

10. Use of compounds of the formula I according to Claim 1 for the purification of integrins by affinity chromatography.

## Revendications

1. Cyclopeptides de formule I
cyclo-(Arg-B-Asp-X-Y) I
dans laquelle
a représente Gly, Ala, -NH-Q-CO- et
X et Y représentent chacun, indépendamment l'un de l'autre, Gly, -HN-Q-CO-, Ala, Asn, Asp, Asp(OR), Arg, Cha, Cys, Gln, Glu, His, Ile, Leu, Lys, Lys(Ac), Lys(AcNH₂), Lys(AcSH), Met, Nal, Nle, Orn, Phe, 4-Hal-Phe, Phg, Pro, Pya, Ser, Thr, Tia, Tic, Trp, Tyr ou Val, les résidus aminoacide cités pouvant également être transformés en dérivés, lorsque X représente Phe, Y ne représentant pas Val,
R représente un groupe alkyle ayant de 1 à 6 atomes de carbone,
Hal représente F, Cl, Br, I,
Q représente un groupe alkylène ayant de 1 à 6 atomes de carbone,
Ac représente un groupe alcanoyle ayant de 1 à 10 atomes de carbone,
dans la mesure où il s'agit de restes d'aminoacides optiquement actifs et de dérivés d'aminoacides, aussi bien les formes D que les formes L étant incluses, ainsi que leurs sels physiologiquement acceptables

2. Enantiomère ou diastéréoisomère d'un composé de formule I selon la revendication 1.

3. (a) cyclo-(Arg-Gly-Asp-D-Lys-Val),
(b) cyclo-(Arg-Gly-Asp-D-Phe-Lys),
(c) cyclo-(Arg-Gly-Asp-D-Phe-Gly),
(d) cyclo-(Arg-Gly-Asp-D-Phe-Phe),
(e) cyclo-(Arg-Gly-Asp-D-Phe-Leu),
(f) cyclo-(Arg-Gly-Asp-Phe-D-Leu).

4. Procédé pour la préparation d'un composé de formule I selon la revendication 1 ou d'un de ses sels, caractérisé en ce qu'on le libère à partir d'un de ses dérivés fonctionnels, par traitement par un agent de solvolyse ou d'hydrogénolyse,
ou en ce que l'on traite par un agent de cyclisation un peptide de formule II
H-Z-OH II
dans laquelle
Z représente -Arg-B-Asp-X-Y-,
-B-Asp-X-Y-Arg-,
-Asp-X-Y-Arg-B-,
-X-Y-Arg-B-Asp- ou
-Y-Arg-B-Asp-X-,
ou un dérivé réactif d'un tel peptide.

5. Procédé pour la préparation de compositions pharmaceutiques, caractérisé en ce que l'on met sous une forme d'administration appropriée un composé de formule I selon la revendication 1 et/ou l'un de ses sels physiologiquement acceptables, conjointement avec au moins un véhicule ou adjuvant solide, liquide ou semi-liquide.

6. Composition pharmaceutique, caractérisée par une teneur en au moins un composé de formule générale I selon la revendication 1 et/ou un de ses sels physiologiquement acceptables.

7. Utilisation de composés de formule I selon la revendication 1, ou de leurs sels physiologiquement acceptables, pour la fabrication d'un médicament destiné à la lutte contre des maladies.

8. Composés de formule I selon la revendication 1, ou sels physiologiquement acceptables de tels composés, pour utilisation dans la lutte contre des maladies.

9. Utilisation de composés de formule I selon la revendication 1, pour la préparation de ligands immobilisés pour la chromatographie sur colonne d'affinité.

10. Utilisation de composés de formule I selon la revendication 1, pour la purification d'intégrines par chromatographie d'affinité.
